Europäisches Patentamt

European Patent Office

Office européen des brevets

(11) Publication number: **0 194 472 B1**

# EUROPEAN PATENT SPECIFICATION

(45) Date of publication of patent specification: **22.07.92**   (51) Int. Cl.5: **C12Q 1/40**, C12Q 1/44

(21) Application number: **86102035.2**

(22) Date of filing: **18.02.86**

(54) Fluorescence polarization assay for macromolecular hydrolases.

(30) Priority: **25.02.85 US 705268**

(43) Date of publication of application:
**17.09.86 Bulletin  86/38**

(45) Publication of the grant of the patent:
**22.07.92 Bulletin  92/30**

(84) Designated Contracting States:
**AT BE CH DE FR GB IT LI NL**

(56) References cited:
**FR-A- 2 255 600
GB-A- 1 381 380
US-A- 3 063 915**

**CLINICAL CHEMISTRY, vol. 19, no. 8, 1973,
pages 838-844, Winston-Salem, NC, US; R.D.
SPENCER et al.: "Design, construction, and
two applications for an automated flow-cell
polarization fluorometer with digital read
out: enzyme-inhibitor (Antitrypsin) assay
and antigen-antibody (insulin-insulin anti-
serum) assay"**

**CLINICAL CHEMISTRY, vol. 31, no. 9, September 1985, pages 1478-1480, Winston-Salem,
NC, US; M. HOFMAN et al.: "Fluorescence
depolarization assay for quantifying alpha-**

**amylase in serum and urine"**

**Fundamentals of Clinical Chemistry, W. B.
Saunden, (Ed.), Philadelphia, Pensylvania,
pp. 625-643 (1976)**

**Hansen et al (1978), Clin. Chem. Vol. 24 pp.
762-768**

**"Textbook of Surgeny", WB. Saunden (Ed.)
Philadelphia, Pensylvania pp. 1284 (1981)**

(73) Proprietor: **ABBOTT LABORATORIES
14th Street and Sheridan Road North St
North Chicago, Illinois 60064(US)**

(72) Inventor: **Shaffar, Mark Raymond
3365 C Beacon Street No.15
North Chicago Illinois 60064(US)**

(74) Representative: **Modiano, Guido et al
MODIANO, JOSIF, PISANTY & STAUB
Modiano & Associati Via Meravigli, 16
I-20123 Milano(IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 194 472 B1

## Description

Technical Field

This inventions relates to a method for conducting a fluorescence polarization assay utilizing reagents, to determine the presence and/or amount of macromolecular hydrolases in a fluid, especially in biological fluid samples such as whole blood, serum, plasma or urine.

Background Art

There are many situations in which it is desirable to analyze for macromolecular hydrolase activity. Known from Fundamentals of Clinical Chemistry, W.B. Saunders (Ed.), Philadelphia, Pennsylvania, pages 625-643 (1976) are methods for measuring hydrolase activity.

In the past, assays for such hydrolases have typically involved the use of a plurality of enzymes and substrates as reagents. Such assays have presented difficulties in that the stability of the various enzymes and substrates, combined in a single reagent solution, is dependent upon conflicting requirements for conditions such as temperature, pH, salinity and the like. For example, the substrates may be more stable in relatively low pH solutions and the enzymes may be more stable in higher pH solutions, such that a serious problem results from the lack of availability of a mutually acceptable environment for the substrate and for the enzymes. Other potential difficulties in conducting such assays are presented by the possibility of cross-reactivity between the various enzymes required for the analysis.

One especially advantageous application for a macromolecular hydrolase assay is the quantitative determination of a polysaccharide hydrolase, such as alpha amylase. Alpha amylase is an enzyme which hydrolyses alpha-glucosidic linkages in starch and related polysaccharides. Several disease states show elevated amylase, including pancreatitis, peptic ulcer, obstruction of the pancreatic duct, pancreatic carcinoma, acute alcohol ingestion or poisoning and salivary gland diseases such as mumps as disclosed in Bradley, et al, Textbook of Surgery, W.B. Saunders (Ed.), Philadelphia, Pennsylvania, page 1294 (1981).

According to one prior art procedure, the activity of alpha amylase is assayed by measuring the degree of light absorption of NADH (reduced nicotinamide adenine dinucleotide) at a wavelength of 340 nm, after reacting a sample with phosphorylase, phosphoglucomutase, glucose-6-phosphate dehydrogenase and a limit dextrin as disclosed in Hanson, et al, Clin.Chem., vol. 24, pages 762-768 (1978).

Another application for a macromolecular hydrolase assay is the quantitative determination of endoproteases such as trypsin. Trypsin is an endoprotease found in the pancreas. Typically, trypsin is assayed by using a chromogenic substrate such as N-$\alpha$-Benzoylarginine ethyl ester (BAEE). Since BAEE is a relatively small synthetic substrate, assays conducted in this manner are not extremely specific.

While small synthetic substrates are relatively simple to make, they tend to lack specificity due to their size. Large synthetic substrates tend to be more specific for a given enzyme, but can be extremely difficult to manufacture. A detailed knowledge of the chemistry of the cleavage of the enzyme is required and, once this knowledge is obtained, the synthesis itself may be complex.

Alternatively, trypsin has been analyzed by placing a sample on a gelatin film. A positive value of trypsin is reported if the gelatin liquifies. This latter method suffers from the drawback that it tends to be merely a qualitative type of analysis. A further assay method applicable to some proteases is the commonly used radioactivity labelling method. A major drawback of this method is that the unreacted substrate must be separated from the reacted substrate before the test results can be taken by measuring the radioactivity.

Known in the art is a fluorescence polarization assay for determining the hydrolase activity of a known amount of trypsin acting on fluorescein isothiocyanate labelled casein in the presence of an unknown amount of trypsin inhibitor which is disclosed by Spencer et al (1973), Clin. Chem 19, 838-844.

Yet another application is the quantitative determination of lipases. In the past, lipases have typically been analyzed by synthetic chromogenic substrate or turbidity clarification methods. Such techniques are not without drawbacks and these methods suffer from problems of stability, purity and characterization of the substrate, as well as problems of specificity and reproducibility.

Accordingly, there presently exists a need for a simple and accurate means to quantitatively analyze fluids for macromolecular hydrolases such as amylases, endoproteases and lipases.

## SUMMARY OF THE INVENTION

The present invention is directed to analytical methods, for conducting a fluorescence polarization assay to quantitatively determine the presence of macromolecular hydrolases selected from amylases and lipases.

The reagents employed in the method of the invention comprise a substrate, capable of being cleaved by the hydrolase being determined, coupled to a fluorophore capable of producing a fluorescence polarization response to plane polarized light.

The methods for synthesizing these reagents comprise coupling a substrate capable of being cleaved by a macromolecular hydrolase with a fluorophore capable of producing a fluorescence polarization response. The general formula for such a substrate can be given by $P-(x-F)_n$, wherein P stands for the macromolecular substrate, which can be chosen from the group consisting of polysaccharides, polypeptides, lipopolypeptides and polynucleotides; wherein x stands for a stable linking group which can be chosen from the group consisting of alkyls, aryls, amides, amines, thioamines, carbonates, thiocarbonates, carbonyls, sulfonates, imides, esters, thioesters, ethers, thioethers; wherein F stands for a fluorophore; and wherein n stands for the number of fluorophores and linking groups on the substrate.

According to the invention, processes for conducting fluorescence polarization assays for macromolecular hydrolases selected from amylase and lipase are provided. These analytical methods comprise preparing a test solution that includes a sample containing the analyte and a substrate, capable of being cleaved by the macromolecular hydrolase being analyzed, which is coupled to a fluorophore moiety capable of producing a fluorescence polarization response. The test solution is then permitted to incubate for a period of time. After incubation, plane polarized light is passed through the resulting solution, and the fluorescence polarization response is detected as an indication of the macromolecular hydrolase in the sample.

Further objects and attendant advantages of the present invention will be best understood with reference to the following detailed description and Examples.

DETAILED DESCRIPTION OF THE INVENTION

This invention is directed to a fluorescence polarization assay for determining the presence of macromolecular hydrolases selected from amylases and lipases, in a sample. The invention provides reagents, and methods for making reagents, used in the assay, as well as methods for conducting the assay. The assay of the present invention can be used for the qualitative or quantitative determination of macromolecular hydrolases, and accordingly, for the purposes of this disclosure, the term "determining" will be understood to apply to both qualitative and quantitative analyses.

The fluorescence polarization assay of the present invention provides many advantages over macromolecular hydrolase assays which have heretofore been known or proposed. Several of these advantages result because only a single substrate is required in the assay system of the invention, while further advantages result from the preferred use of a natural, rather than a synthetic substrate.

Since only one substrate is required in the assay system of the present invention, problems attendant to stabilization of the assay system are minimized. As has been discussed above, in other assay systems the several substrates and enzymes used often have mutually incompatible requirements for stability. A concommittant advantage is that only a single substrate need to be made for use in the assay system.

The assay of the present invention preferably uses a natural, rather than a synthetic, substrate. The preparation of a natural substrate is a relatively simple task, because it is not necessary to chemically determine the structure of a synthetic substrate that will be specific for the macromolecular hydrolase being determined, nor is it necessary to map out and execute a complex synthetic procedure directed to producing such a substrate. An important result of using a single substrate, which is itself easily prepared, is relatively low reagent cost. Still further, assays conducted in accordance with the present invention are highly sensitive, and extremely reproducible. Natural substrates are also especially advantageous because they tend to produce even better assay specificity by comparison with synthesized materials.

The assay of the present invention operates according to the principles of fluorescence polarization. According to these principles, if a fluorophore is excited by a plane-polarized beam of light, it will in turn emit polarized light. The degree of polarization of the emitted light will be inversely related to how much the molecule containing the fluorophore has rotated between the time of excitation and emission. The time it takes for a molecule to rotate through an angle of approximately 68.5 degrees has been defined as the rotational relaxation time of the molecule. The rotational relaxation time is relatively small (on the order of about one nanosecond) for small molecules (e.g., such as fluorescein), and large (on the order of about 100 nanoseconds) for large molecules (e.g., such as immunoglobulins). The rotational relaxation time of a molecule is primarily dependent on its volume, so that the observed polarization of its fluorescence in solution gives a direct indication of its size.

Still another advantage of the assay of the present invention is that it is a homogenous assay, i.e., it is not necessary to separate the cleaved products from the unreacted substrate before taking the fluorescence polarization reading.

According to the present invention, macromolecular hydrolases can be analyzed by providing a substrate capable of being cleaved by the macromolecular hydrolase being determined, where the substrate is labelled with a fluorophore. By sequentially exciting the reaction mixture with vertically and then horizontally polarized light and analyzing only the vertical component of the emitted light, the polarization of fluorescence in the reaction mixture can be determined very accurately. When the substrate is intact, the fluorophore is part of a very large molecule, so the fluorescence polarization response yields a high value. When the substrate has been cleaved by the hydrolase enzyme, the fluorophore is part of a smaller molecule, and the fluorescence polarization response attributable to that fluorophore moiety will be substantially reduced. If much enzyme is present, the rate of fluorescence depolarization will be substantial, while if little or no enzyme is present, the macromolecular substrates will be cleaved relatively slowly or not all, and the rate of fluorescence depolarization will be correspondingly slow.

The principles of the present invention are applicable to the broad category of compounds known as macromolecular hydrolases, which includes, but is not limited to: alpha and beta amylases; lipases; esterases; pectinases; nucleotidase; laminarinase; dextranase; polygalacturonase; chitinase; lysozyme; neuraminidase; hyaluronidase; nucleosidases; aminopeptidase; carboxypeptidases; glutamylcarboxypeptidase; proinsulinase; insulinase; rennin; pepsin; trypsin; chymotrypsin; pancreatopeptidase; cathepsin; papain; chymopapain; ficin; thrombin; plasmin; subtilopeptidase; aspergillopeptidase; streptococcus peptidase; streptokinase; clostridiopeptidase; bromelain; keratinase; urokinase; enteropeptidase; kallikrein, apopyridoxalenzyme hydrolase; and pectate lyase. The broad classes of macromolecular hydrolases that are of particular interest for the purposes of this invention include the amylases and the lipases, which are exemplified herein.

A wide variety of fluorophores can be used in the method of the present invention. As previously indicated, the choice of the fluorophore will depend upon the particular macromolecular hydrolase to be determined and reagent system employed. Representative of the classes of fluorophores useful are fluoresceins, rhodamines, flavins, coumarins, naphthalenes, acridines, anthracenes, polynuclear fused hydrocarbons, stilbenes, anthranilic acids, aminostyrylpyridines, quinolines, salicylic acids, cyanines, oxonols, phenanthidines, fluorescamines, as well as derivatives and salts thereof. Illustrative of specific fluorophores that can be used include, for example, eosin, rhodamine, aminonapthalene sulfonate, acriflavin, fluorescein, dihydroxybenzoic acid, hydroxyquinoline, NADH, riboflavin, brilliant sulfaflavin, quinine, naphtholsulfonic acid, thioflavin, coumarin, acridine orange, 8-anilino-1-naphthalene sulfonic acid, oxazine, umbelliferone, acridine, resorufin, and derivatives and salts thereof. In the presently preferred embodiments, fluoresceins are used; specifially 4,6-dichlorotriazin-2-(yl)amino fluorescein (DTAF) and fluorescein isothiocyanate (FITC) have been found to be especially effective and advantageous.

Illustrative of the method of the invention are those wherein the substrate comprises amylase, gelatin, hemoglobin, casein or lipoprotein and is coupled to a fluorescein moiety. Illustrative fluorescein moieties to be used in such method are 4,6-dichlorotriazin-2-(yl)aminofluorescein, a derivative of 4,6-dichlorotriazin-2-(yl)-aminofluorescein, fluorescein isothiocyanate or a derivative of fluorescein isothiocyanate.

The selection of the fluorophore to be used, as will be well understood by those skilled in the art, will depend upon such factors as: (1) the excitation and emission wavelengths of the fluorophore and of the instrument employed; (2) the linking functionality between the fluorophore and the substrate; (3) the rotational relaxation time of the free label; (4) the quantum yield; and (5) the extinction coefficient.

In addition, the proper selection of wavelengths of excitation and emission of the fluorophore is important for the reduction of interference from various components in the sample under analysis, such as hemoglobin and bilirubin. Accordingly, the fluorophore should be chosen such that its excitation and emission wavelengths do not overlap the absorbance spectrum of such substances as hemoglobin and bilirubin.

The linking functionality affects the choice of fluorophore in that certain substrates have functional groups known to react with certain fluorophores. For example, DTAF reacts strongly with the amine moiety on lysine groups; accordingly, when lysine groups are readily available on the substrate, DTAF may be the fluorophore of choice. Other examples will be readily ascertainable to those skilled in the art. Sometimes connecting groups, such as aliphatic chains, can be used to link the respective linking functionality of the fluorophore and the substrate. A list of types of exemplary linking groups has been previously set forth.

The rotational relaxation time is calculated by the Perrin equation, which is well known to those skilled in the art. This is an important factor because the accuracy of the assay will depend upon detecting differences in fluorescence polarization response over a period of time. If the cleaved fluorophore substrate produces a polarization response not appreciably different from that produced by the intact fluorophore-substrate, then it will be difficult to detect a significant depolarization response. The quantum yield and extinction coefficient of the fluorophore are important because they affect the minimum degree of substitution of the fluorophore onto the substrate, so that a sufficient fluorescence intensity can be observed for a working concentration of substrate. If the quantum yield and extinction coefficient are poor, then an insufficient fluorescence intensity for an accurate measurement of the fluorescence polarization of a solution may be observed. A poor quantum yield necessitates a high degree of substitution on the substrate.

Substrates should be chosen with a view toward how well they are cleaved by the macromolecular hydrolase being analyzed. It is important that the cleavage occur such that the size of the substrate will be considerably reduced by the hydrolase in a relatively short period of time. The presently preferred substrate for an assay for alpha amylase in accordance with the invention, for example, is potato amylose, which produces good results when coupled to the fluorophore moieties DTAF and FITC described supra. Other substrates that have produced good results in this assay are amylopectin, potato starch, Litner's soluble starch, and Maltrin® 040, 050, 100, 250, 500 and 550, obtainable from the Grain Processing Corporation, Muscatine, Iowa. Other useful substrates are starches such as wheat starch, rice starch, corn starch and dextrin.

A suitable enzyme calibration procedure involves preparing a series of solutions containing varying amounts of enzyme, and then obtaining an enzyme activity value from assay of these solutions by a reference method. The calibrated enzyme solutions are then analyzed by the fluorescence polarization procedure set forth above. The polarization values are plotted against the enzyme activity values to construct a standard curve.

The assay parameters are optimized by balancing the variables of the procedure. The sample size incubation parameters of the assay are optimized by balancing the variables of assay span (expressed as millipolarization between the lowest and the highest calibrator), sample matrix interference, assay throughput, incubation time and precision. The presently preferred optimal sample size is twenty-five microliters per two milliliters of reaction volume. An optimal throughput is obtained by keeping the incubation time to about five minutes or less. For precision with a coefficient of variation of less than 5%, an assay span of greater than 50 millipolarization units is preferred. The substrate concentration in the reagent mixture is optimized by balancing the assay span, the photometer intensity and the sample matrix interference.

An assay for amylase conducted in accordance with the preferred procedure set forth herein has proven highly accurate. A correlation study was performed comparing a commercially available amylase test (Agent®, from Abbott Laboratories, North Chicago, Illinois) to the results obtained from the preferred procedure, using 36 patient samples with varying levels of amylase. The correlation study gave good results, demonstrating the accuracy of an amylase assay conducted in accordance with the invention.

Of course, it should be understood that the presently preferred procedures and assay parameters are set forth only by way of illustration and not limitation, and can be greatly varied, as will be apparent to one skilled in the art, to suit particular applications. Accordingly, although the presently most preferred assay conditions involve an assay temperature at or about 35°C, incubation times at or about 5 minutes, a pH at or about 7.5 and a substrate concentration at or about 50 micrograms per millimeter, these conditions can be varied widely. Thus, for example, the assay temperature can be kept within the range of about 15°C to about 45°C; the incubation time can be selected from between about a second to about several hours, depending upon the desired sensitivity of the assay (although incubation times selected from between about 1 minute to about 1 hour are preferred); the pH can be maintained in a range from about 3 to about 11, depending upon the enzyme activity at the specified pH; and the substrate concentration can be in the range from about 0.01 nanograms/ml to about 300 mg/ml in the final test solution, depending upon the solubility and quantum yield of the fluorophore.

Further, the preferred fluorescence depolarization assay of the invention can be varied, for example, by testing the fluorescence polarization response not necessarily at intervals commencing from immediately after the time of incubation. For example, a single reading can be taken at a time after incubation and compared with a reading from a control containing substrate but no enzyme. Yet another technique may be configured wherein sample and reagent are combined and preincubated for a period of time before the initial reading is taken. The solution can then be incubated further for a set interval of time before the second reading is taken. The fluorescence polarization response between the two time points is then used.

It is therefore intended that the foregoing detailed description and the following Examples be regarded as illustrative rather than limiting, and that the scope of the invention be defined solely by the appended claims, including all equivalents thereof.

Example 1

The following Example sets forth a preferred procedure, in order to illustrate one method of making a substrate coupled to a fluorophore moiety according to the invention. One gram of potato amylose is dissolved in 10 ml of dimethyl sulfoxide (DMSO). Two drops of pyridine, 700 microliters of dibulytindilaurate and 10 mg of fluorescein isothiocyanate (FITC) are added. The solution is heated to about 100°C for about 3 hours, and then allowed to cool. After cooling, the labelled substrate is purified by adding 100 ml of ethanol (EtOH) to precipitate the starch. The mixture may be further cooled to facilitate precipitation. The precipitated starch is filtered and redissolved in 10 ml of DMSO, and then reprecipitated with 100 ml of ethanol. Finally, the precipitated starch is filtered, dried and the product stored.

According to a presently preferred procedure for conducting the assay, three reagents are prepared, which, for convenience, will be referred to as A, B and C: A = 300 mg/ml fatty acid-free bovine serum albumin; B = 10 mg/ml fluorescein labelled amylose in 85% DMSO with 0.1 M NaCl; and C = 3.6 M NaCl. A buffer solution, hereinafter referred to as "TDx® buffer," comprises 0.1 M sodium phosphate buffer with 0.01% bovine gamma globulin and 0.10% sodium azide, at pH 7.5. It should also be noted that in this preferred embodiment, the assay can be advantageously performed on an Abbott TDx Fluorescence Polarization Analyzer. Twelve and one-half microliters of C reagent are mixed with 25 microliters of A reagent into 962.5 ml of TDx buffer. Plane polarized light is passed through the mixture and the horizontal and vertical intensities are read. Then 12.5 microliters of C reagent are added to the solution, along with 25 microliters of B reagent, 20 microliters of sample and an additional 942.5 microliters of TDx buffer, to provide a test solution. The test solution is permitted to incubate for 5 minutes at 35°C. Plane polarized light is passed through the solution, and the fluorescence polarization is measured. The final polarization response is calculated and compared to polarizations from a calibration curve.

Example 2

Three grams of amylose from sweet potato were added to a solution of approximately 2 mg of DTAF in approximately 100 ml of TDx buffer. The amylose did not completely dissolve. After periods of 5 and 30 minutes, 20 microliters were removed and diluted to 1 ml. Then 20 microliters of the diluted mixture were further diluted in 1 ml of buffer. This solution was added to a cuvette and the fluorescence intensity and net millipolarization were read at gain 5 on the Abbott TDx Fluorescence Polarization Analyzer. Five minutes after the coupling reaction was initiated, the fluorescence polarization was observed to rise, indicating a coupling of the DTAF to amylose. Ten microliters of a 10 milligram per milliliter bacterial amylase solution were then added to the cuvette containing the DTAF coupled amylose. The polarization then immediately dropped due to the hydrolysis of the amylose into small fragments, thus demonstrating the principle of this technique.

Example 3

The substrate from Example 2 was further prepared by adding approximately 100 ml of EtOH and 100 ml of acetone to precipitate the fluorescein labelled amylose. The material was then filtered in a buchner funnel, using approximately 50 ml EtOH to wash away free DTAF. The amylose was dried and resuspended in water. The solution was then filtered to remove insoluble amylose.

Example 4

To 1 ml of TDx buffer, 90 microliters of saturated NaCl solution, 25 microliters of substrate from Example 3 and 25 micrograms of sample were added. The solutions were mixed and incubated at 35°C. At various incubation times the fluorescence polarization was read on the TDx analyzer. The samples used were Sigma normal and elevated enzyme. These samples contained approximately 1700 and 4500 Sigma units per liter of amylase, respectively. The following data were obtained:

| INCUBATION TIMES | | | |
|---|---|---|---|
| | 2 minutes | 7 minutes | 12 minutes | 18 minutes |
| no enzyme | 214 mp | 214 mp | 213 mp | 212 mp |
| Sigma normal | 213 mp | 193 mp | 179 mp | 165 mp |
| Sigma elevated | 202 mp | 167 mp | 150 mp | 138 mp |

The data demonstrates that the porcine amylase (in the Sigma controls) cleaved the substrate.

Example 5

One gram of sweet potato amylose was dissolved in 10 milliliters of dimethyl sulfoxide (DMSO). Two drops of pyridine, 700 microliters of dibutyltindilaurate and 10 mg of FITC were added to the solution. When dissolved, the solution was heated at 100°C on a steam bath for three hours. After cooling, 100 ml of ethanol were added to precipitate the amylose. The precipitated amylose was filtered using a sintered glass funnel. The unreacted FITC in the ethanol filtrate was discarded. The labelled amylose was then redissolved in DMSO and reprecipitated two more times.

Example 6

Two ml of lipoprotein solution (Miles Laboratories, Inc. code 82-018) were mixed with 2 ml of DMSO. Forty microliters of a 100 mg/ml solution of FITC in DMSO were then added. Two drops of pyridine were added, along with 200 ml of dibutyltin dilaurate. The solution was placed on a steam bath for two hours.

After two hours, 10 microliters of solution were diluted in 1 ml of DMSO. This was further diluted 1/100 into TDx buffer. The millipolarization of the solution was found to be 183.

Example 7

The fluorescein-labelled lipoprotein substrate from Example 6 was diluted into 1 ml of TDx buffer, and various concentrations of lipoprotein lipase were added. The solution was then incubated at 35°C for 5 minutes before the fluorescence polarization was determined. A decrease in concentration proportional to enzyme concentration was observed. The resulting data follows.

| Lipase Added | MP |
|---|---|
| none | 180 |
| 65 units | 122 |
| 163 units | 93 |

It will be apparent that various modifications and changes can be made in the specific embodiments of the invention described herein, without departing from the spirit and scope thereof, as defined solely in the following claims.

**Claims**

1.  A process for determining the activity of a macromolecular hydrolase selected from amylase and lipase, comprising the steps of:

    a) preparing a test solution comprising a sample suspected of containing the macromolecular hydrolase, and a fluorophore-labelled substrate capable of being cleaved by the macromolecular hydrolase;

    b) incubating the test solution for a period of time;

    c) passing plane polarized light through the test solution of step b); and

    d) detecting the fluorescence polarization response from step c) as an indication of the macromolecular hydrolase activity in the sample.

2.  The process of Claim 1 wherein the substrate comprises amylose, gelatin, hemoglobin, casein or lipoprotein and is coupled to a fluorescein moiety.

3.  The process of Claim 2 wherein the substrate is coupled to 4,6-dichlorotriazin-2-(yl) aminofluorescein, a derivative of 4,6-dichlorotriazin-2-(yl) aminofluorescein, fluorescein isothiocyanate or a derivative of fluorescein isothiocyanate.

**Revendications**

1.  Un procédé pour déterminer l'activité d'une hydrolase macromoléculaire choisie parmi l'amylase et la lipase, comprenant les étapes suivantes :

a) préparation d'une solution de test comprenant un échantillon suspecté comme contenant l'hydrolase macromoléculaire, et un substrat marqué par un fluorophore susceptible d'être clivé par l'hydrolase macromoléculaire ;

b) incubation de la solution de test pendant une période de temps ;

c) passage de la lumière polarisée dans un plan à travers la solution de test de l'étape b) ; et

d) détection de la réponse de polarisation de la fluorescence dans l'étape c) comme une indication de l'activité de l'hydrolase macromoléculaire dans l'échantillon.

2. Le procédé selon la revendication 1, selon lequel le substrat comprend l'amylose, la gélatine, l'hémoglobine, la caséine ou la lipoprotéine et est couplé à une moitié fluorescéine.

3. Le procédé selon la revendication 2, selon lequel le substrat est couplé à la 4,6-dichlorotriazine-2-(yl)-aminofluorescéine, un dérivé de la 4,6-dichlorotriazine-2-(yl)aminofluorescéine, l'isothiocyanate de fluorescéine ou un dérivé de l'isothiocyanate de fluorescéine.

**Patentansprüche**

1. Verfahren zum Bestimmen der Ativität einer aus Amylase und Lipase ausgewählten makromolekularen Hydrolase, welches die folgenden Stufen umfaßt:

a) Herstellen einer Testlösung, welche eine Probe enthält, von welcher vermutet wird, daß sie die makromolekulare Hydrolase enthält, und welche Testlösung ein mit einem Fluorophor markiertes Substrat enthält, welches von der makromolekularen Hydrolase gespalten werden kann;

b) Inkubieren der Testlösung während einer Zeitspanne;

c) Hindurchführen von in einer Ebene polarisiertem Licht durch die Testlösung der Stufe b); und

d) Feststellen der Fluoreszenz-Polarisationsantwort aus der Stufe c) als Hinweis auf die Aktivität der makromolekularen Hydrolase in der Probe.

2. Verfahren nach Anspruch 1, wobei das Substrat Amylose, Gelatine, Haemoglobin, Casein oder Lipoprotein umfaßt und an einen Fluoresceinrest gekuppelt ist.

3. Verfahren nach Anspruch 2, wobei das Substrat an 4,6-Dichlortriazin-2(yl)-aminofluorescein, ein Derivat von 4,6-Dichlortriazin-2-(yl)-aminofluorescein, Fluorescein-isothiocyanat oder ein Derivat von Fluorescein-isothiocyanat gekuppelt ist.